**Europäisches Patentamt**

**European Patent Office**

**Office européen des brevets**

(19)

(11) Veröffentlichungsnummer: **0 085 783**
**B1**

(12) **EUROPÄISCHE PATENTSCHRIFT**

(45) Veröffentlichungstag der Patentschrift:
**12.03.86**

(51) Int. Cl.⁴: **A 61 B  6/12**, G 03 C  5/16

(21) Anmeldenummer: **82111911.2**

(22) Anmeldetag: **22.12.82**

(54) **Fotografisches Verfahren zum Herstellen eines Angiogramms.**

(30) Priorität: **05.02.82  DE 3204037**

(43) Veröffentlichungstag der Anmeldung:
**17.08.83 Patentblatt 83/33**

(45) Bekanntmachung des Hinweises auf die Patenterteilung:
**12.03.86 Patentblatt 86/11**

(84) Benannte Vertragsstaaten:
**AT BE CH DE FR GB IT LI LU NL SE**

(56) Entgegenhaltungen:
**DE - A - 2 530 315**
**DE - C - 882 752**
**US - A - 4 204 225**
**US - A - 4 204 226**

**TOSHIBA REVIEW, INTERNATIONAL EDITION,
Juli-August 1969, No 43, Verlag: Tokyo Shibaura Electric
Co., Ltd. M. NAKASHIKA; H. WATANABE, T.OKUBO, K.
NISHIO, Y. WATASE " Latest Diagnostic System for
Circulartory Organs" Seiten 24-29**

**Die Akte enthält technische Angaben, die nach dem
Eingang der Anmeldung eingereicht wurden und die
nicht in dieser Patentschrift enthalten sind.**

(73) Patentinhaber: **STB Strahlentechnische Bilddiagnostik
Heinz Fleck, Haidkamp 95, D-2080 Pinneberg (DE)**

(72) Erfinder: **Lohse, Karl-Heinz, Im Bans 20a,
D-2080 Pinneberg (DE)**

(74) Vertreter: **Fleck, Thomas, Dr.Dipl.-Chem. et al,
Patentanwälte Raffay, Fleck & Partner Postfach 32 32 17,
D-2000 Hamburg 13 (DE)**

## Beschreibung

Die Erfindung betrifft ein fotografisches Verfahren nach dem Oberbegriff des Anspruchs 1.

Dieses Verfahren ist in der angiographischen Praxis gebräuchlich und bietet den Vorteil, dass in dem Betrachtungsbild die mit Kontrastmittel kenntlich gemachten Gefäss-Strukturen stark hervorgehoben sind. Theoretisch könnte der gesamte übrige Bildinhalt verschwinden; praktisch verbleiben aber wegen unvermeintlicher Verschiedenheiten der Aufnahmebedingungen, insbesondere wegen nicht zu unterdrückender Bewegungen des lebenden Gefässbereiches, schwache reliefartige Andeutungen von anderen Organstrukturen oder Knochen. Dadurch wird bei der Betrachtung die Orientierung erleichtert.

Meist wird nach dem Einleiten des Kontrastmittels eine ganze Reihe von Aufnahmen in bestimmten Zeitabständen, z.B. alle 0,5 Sekunden, hergestellt, die alle unter fotografischer Subtraktion der Leeraufnahme zu Betrachtungsbildern verarbeitet werden, so dass man auf diesen die Weiterförderung des Kontrastmittels und dabei etwa auftretende Hindernisse im Gefässsystem erkennen kann. Da somit zumindest zwei, meist aber noch erheblich mehr, Angiogramme gemacht werden, ist die Strahlenbelastung des untersuchten Organismus beträchtlich. Dies gilt besonders dann, wenn in der vielfach geübten Weise gern etwas überexponiert wird, um mit Sicherheit brauchbare Angiogramme zu erhalten. Dennoch sind diagnostisch interessante Einzelheiten in der Betrachtungsaufnahme oft nur schwer oder überhaupt nicht erkennbar, weil die abbildenden Dichteunterschiede (Schwärzungsunterschiede) zu gering sind.

Die Erfindung geht deshalb von der Aufgabe aus, ein fotografisches Verfahren zu schaffen, das mit erheblich geringeren Strahlendosen zu Betrachtungsbildern hoher Qualität führt.

Nach der Erfindung wird diese Aufgabe gelöst mit einem fotografischen Verfahren nach dem Anspruch 1.

Nach dem erfindungsgemässen Verfahren kann man ohne weiteres den Dichtebereich um 2 bis 4 Einheiten transponieren, also die zur Aufnahme verwendete Strahlendosis mit dem Faktor $10^2$ bis $10^4$ verringern. Auch die Einsparung an Kontrastmittel ist beträchtlich. Bei den normalerweise hohen Preisen üblicher Kontrastmittel ergeben sich dadurch hohe Kosteneinsparungen; ausserdem wird die Belastung des Organismus mit Kontrastmittel stark verringert.

Es versteht sich, dass man bei dem erfindungsgemässen Verfahren den für die direkte Betrachtung ungeeigneten Dichtebereich nicht zu nahe bei 0 wählen darf, weil man dort in den Anfangsbereich der Schwärzungskurve fotografischer Schichten kommt und die Anwendbarkeit fotografischer Verfahren ihr natürliches Ende findet. Man kann aber ohne weiteres Bereiche sehr geringner Dichte (Schwärzung) wählen, die einer erheblichen Verringerung der Expositionszeit um mehrere Grössenordnungen entsprechen. Im erfindungsgemässen Fall wird der für die direkte Betrachtung ungeeignete Dichtebereich zu 0,2 ... 1,5 gewählt. Dabei ergibt sich schon eine Herabsetzung der Expositionszeit auf etwa 1/10.

Besonders vorteilhaft ist es, wenn man den zur direkten Betrachtung ungeeigneten Dichtebereich in einen grösseren Betrachtungs-Dichtebereich transponiert; das ist durch Verwendung von hartem fotografischem Material ohne weiteres möglich.

Das erfindungsgemässe Verfahren erfordert nur einfache und leicht mit hoher Genauigkeit und Reproduzierbarkeit durchführbare Schritte, die alle in der fotografischen Technik an sich bekannt sind. Besonders einfach ist das erfindungsgemässe Verfahren, wenn die Aufnahmen wie üblich Dias sind und man zum Herstellen des Betrachtungsbildes unter fotografischer Subtraktion in bekannter Weise ein Umkehr-Dia der Leeraufnahme herstellt und das Umkehr-Dia und das Aufnahme-Dia registerhaltig übereinanderlegt und zu dem Betrachtungsbild kopiert, ggf. unter Änderung des Abbildungsmassstabes. Das entspricht der auch bisher meist üblichen Arbeitsweise, so dass keine neuen Arbeitstechniken eingeübt werden müssen. Man erkennt ohne weiteres, dass die beschriebenen Arbeitsschritte auch ohne weiteres mechanisch und automatisch ausgeführt werden können, so dass es keine grundsätzlichen Schwierigkeiten bereitet, Vorrichtungen zu schaffen, in denen das Herstellen des Umkehr-Dias, das Übereinanderlegen der beiden Dias und das Herstellen des Betrachtungsbildes selbsttätig maschinell zum Ablauf gebracht wird. Diese automatische Arbeitsweise ist naturgemäss besonders vorteilhaft. Dies gilt um so mehr als angiographische Verfahren leicht auf bestimmte häufig zu untersuchende Gefässbereiche standardisiert werden können, beispielsweise auf Herz, Gehirn, Gallenblase und dergleichen. Es liegen dann für jeden derartigen Bereich bei allen Untersuchungen weitgehend gleiche Aufnahmebedingungen vor, so dass es sehr leicht ist, durch Ausprobieren die Verfahrens-Parameter (Expositionszeit, Konstanten der fotografischen Entwicklungsvorgänge) so zu bestimmen, dass für eine ganze Reihe von Untersuchungen optimale Ergebnisse erzielt werden.

Zweckmässigerweise verwendet man zum Herstellen des zuletzt erwähnten Umkehr-Dias ein fotografisches Material mit einer bei 1 liegenden Gradation ($\gamma = 1$) und zum Herstellen des Betrachtungsbildes ein Material steilerer Gradation; auf diese Weise gelingt es, einen verhältnismässig schmalen Schwärzungsbereich, beispielsweise 0,2 bis 1,5, in welchem die gewünschte Information enthalten ist, auf einen entsprechend grösseren Schwärzungsbereich, beispielsweise 0,5 bis 2,5, in welchem eine direkte Betrachtung besonders leicht möglich ist, zu transponieren. Zum Herstellen des Umkehr-Dias ist dagegen eine Gradation bei 1 erforderlich, damit die Maskierung des Aufnahme-Dias mit dem Umkehr-Dia die nicht interessierenden Strukturen möglichst weitgehend auslöscht. Es versteht sich, dass man natürlich auch für eine schwache Darstellung dieser Strukturen sorgen kann, indem man zur Herstellung des Umkehr-Dias ein Material mit einer etwas von 1 verschiedenen Gradation verwendet und/oder geringfügige Abweichungen von der Registerhaltigkeit der beiden Dias bewusst einführt. Derartige Techniken sind an sich bekannt.

Vorzugsweise verwendet man als fotografisches Material steilerer Gradation ein solches mit einer Gradation im Bereich von etwa 3 bis 8. In den meisten Fällen kommt man mit einer Gradation von etwa 3 gut aus. Bei höheren Gradationen müssen Exponierung und Verarbeitung des fotografischen Materials mit entsprechend höherer Genauigkeit erfolgen.

Die bei Verwendung fotografischen Materials steilerer Gradation für die Herstellung des Betrachtungsbildes erzielte Kontrast-Expansion macht es möglich, Strukturen zu erkennen, die in einer normalen Aufnahme wegen geringer Dichteunterschiede nicht wahrnehmbar sind. Auf diese Weise gelingt es beispielsweise, zuverlässige Aussagen über die Beschaffenheit von Gefässwänden zu gewinnen. Es versteht sich, dass man für solche und ähnliche Sonderaufgaben die Kontrast-Expansion höher wählen kann als bei der Abbildung von fotografisch differenzierteren Strukturen; so kann man beispielsweise mit Vorteil zunächst mit geringer Kontrast-Expansion die Gefässe abbilden und dann mit höheren Kontrast-Expansionen weitere Bilder herstellen, die über bestimmte Feinstrukturen Aufschluss geben. In jedem Fall kann man in bekanntenr Weise den ausgezogenen und im Kontrast expandierten Dichtebereich durch Verändern der Belichtung beim Herstellen des Betrachtungsbildes variieren.

## Patentansprüche

1. Fotografisches Verfahren zum Herstellen eines Angiogramms mittels fotografischer Subtraktion, bei dem eine Umkehraufnahme einer röntgenografischen Leeraufnahme ohne Kontrastmittel und eine zweite anschliessend unter Verwendung von Kontrastmittel hergestellte Röntgenaufnahme registerhaltig aufeinandergelegt werden und davon zum Herstellen eines Betrachtungsbildes eine fotografische Aufnahme gefertigt wird, dadurch gekennzeichnet, dass die in den Röntgenaufnahmen enthaltene Information im Dichtebereich (Schwärzungsbereich) von 0,2 bis 1,5 liegt, so dass sie bei direkter Betrachtung nicht erkennbar ist, und dass die fotografische Subtraktion derart ausgeführt wird, dass die im resultierenden Betrachtungsbild enthaltene Information in einem für die direkte Betrachtung geeigneten Dichtebereich liegt.

2. Verfahren nach Anspruch 1, dadurch gekennzeichnet, dass man den ungeeigneten Dichtebereich in einen grösseren Betrachtungs-Dichtebereich transponiert.

3. Verfahren nach einem der vorhergehenden Ansprüche, bei dem die Aufnahmen Dias sind, dadurch gekennzeichnet, dass man zum Herstellen des Betrachtungsbildes unter fotografischem Subtrahieren in bekanntnr Weise ein Umkehr-Dia der Leeraufnahme herstellt und das Umkehr-Dia und das Aufnahme-Dia registerhaltig übereinanderlegt und zu dem Betrachtungsbild kopiert, ggf. unter Änderung des Abbildungsmassstabes.

4. Verfahren nach Anspruch 3, dadurch gekennzeichnet, dass das Herstellen des Umkehr-Dias, das Übereinanderlegen der beiden Dias und das Herstellen des Betrachtungsbildes selbsttätig maschinell zum Ablauf gebracht wird.

5. Verfahren nach einem der Ansprüche 3 oder 4, dadurch gekennzeichnet, dass man zum Herstellen des Umkehr-Dias ein fotografisches Material mit einer bei 1 liegenden Gradation und zum Herstellen des Betrachtungsbildes ein Material steilerer Gradation verwendet.

6. Verfahren nach Anspruch 5, dadurch gekennzeichnet, dass man als Material steilerer Gradation ein Material mit einer Gradation im Bereich von etwa 3 bis 8 verwendet.

## Claims

1. Photographic process for the production of an angiogram by means of photographic substraction, wherein a reversal photograph of an X-ray blank photograph without contrast medium and a second X-ray photograph, subsequently produced using contrast medium, are placed in register one upon the other and a photographic record is made therefrom for the purpose of producing a viewing picture, characterized in that the information contained in the X-ray photographs lies in the density range from 0.2 to 1.5, so that it cannot be recognised when directly viewed, and that the photographic substraction is carried out in such a manner that the information contained in the resulting viewing picture lies in a density range suitable for direct viewing.

2. Process according to Claim 1, characterized in that the unsuitable density range is transposed into a larger viewing density range.

3. Process according to one of the preceding Claims, wherein the photographs are diapositives, characterized in that, for the production of the viewing picture by photographic substraction in known manner, a reversal diapositive of the blank photograph is produced and the reversal diapositive and the photographic diapositive are placed in register one upon the other and are copied to give the viewing picture, perhaps with a change to the scale of representation.

4. Process according to Claim 3, characterized in that the production of the reversal diapositive, the placing of the two diapositives one upon the other and the production of the viewing picture are carried out mechanically in automatic sequence.

5. Process according to one of Claims 3 or 4, characterized in that the reversal diapositive, a photographic material having a gradation close to one is used and for the production of the viewing picture a material of a steeper gradation is used.

6. Process according to Claim 5, characterized in that as material of steeper gradation a material having a gradation in the range from approximately 3 to 8 is used.

## Revendications

1. Procédé photographique de production d'un angiogramme par soustraction photographique, dans lequel une épreuve négative d'une exposition à vide radiographique sans substance de contraste et une seconde radiographie produite ensuite en utilisant une substance de contraste sont superposées l'une sur l'autre à repérage et dans lequel réalisé une épreuve photographique de ces radiographies pour produire une épreuve à observer, caractérisé en ce que l'information contenue dans les radiographies se trouve dans la zone de densité (zone de noircissement) de 0,2 à 1,5 de manière à pourvoir être reconnue lors de l'observation directe et que la soustraction photographique est réalisée de manière à ce que l'information contenue dans la radiographie finale à observer se trouve dans une zone de densité convenant à l'observation directe.

2. Procédé selon la revendication 1, caractérisé en ce que l'on transpose la zone de densité ne convenant pas en une zone de densité plus élevée pour l'observation.

3. Procédé selon l'une ou l'autre des revendications précédentes dans lequel les radiographies sont des diapositives, caractérisé en ce que l'on produit un négatif de l'exposition à vide pour produire une radiographie à observer de manière connue, par soustraction photographique, que l'on superpose le négatif et la diapositive de la pose à repérage et qu'on les copie pour en faire la radiographie à observer, éventuellement en modifiant le grandissement.

4. Procédé selon la revendication 3, caractérisé en ce que le déroulement de la production du négatif, de la superposition des deux diapositives et de la production de la radiographie à observer est machinel et automatique.

5. Procédé selon l'une des revendications 3 ou 4, caractérisé en ce que l'on utilise un matériel photographique d'une gradation de l'ordre de 1 pour produire le negatif et un matériel d'une gradation plus forte pour produire la radiographie à observer.

6. Procédé selon la revendication 5, caractérisé en ce que l'un utilise comme matériel de plus forte gradation un matériel d'une gradation de l'ordre d'environ 3 à 8.